(19) Europäisches Patentamt
European Patent Office
Office européen des brevets

(11) Veröffentlichungsnummer: **0 090 196**
**A1**

# (12) EUROPÄISCHE PATENTANMELDUNG

(21) Anmeldenummer: **83102110.0**

(22) Anmeldetag: **04.03.83**

(51) Int. Cl.³: **C 07 C 63/00**, C 07 C 51/09, C 07 D 307/88

(30) Priorität: **25.03.82 DE 3211002**

(43) Veröffentlichungstag der Anmeldung: **05.10.83 Patentblatt 83/40**

(84) Benannte Vertragsstaaten: **AT BE CH DE FR GB IT LI LU NL SE**

(71) Anmelder: **DYNAMIT NOBEL AKTIENGESELLSCHAFT, Patentabteilung Postfach 1209, D-5210 Troisdorf, Bez. Köln (DE)**

(72) Erfinder: **Zoche, Günter, Dr., Königsheimstrasse 13, D-5300 Bonn 3 (DE)**
Erfinder: **Petersen, Egon-Norbert, Dr., Im Immenthal 42, D-5205 Neunkirchen-Seelscheid 1 (DE)**

(54) **Verfahren zur Herstellung von Dibenzyl-2-carbonsäuren und in 3-Stellung substituierten Phthaliden sowie dabei anfallende neue Benzylderivate.**

(57) Ein Verfahren, bei dem, je nach Reaktionsführung, Dibenzyl-2-carbonsäuren oder in 3-Stellung durch einen, gegebenenfalls substituierten, Benzylrest substituierte Phthalide erhalten werden. Einige der nach dem Verfahren hergestellten Verbindungen sind bisher unbekannt gewesen.

EP 0 090 196 A1

ACTORUM AG

Troisdorf, den 10.3.1982
OZ: 82021 (4122) Dr.Sk/Ce

DYNAMIT NOBEL AKTIENGESELLSCHAFT
Troisdorf, Bez. Köln

**Verfahren zur Herstellung von Dibenzyl-2-carbonsäuren und in 3-Stellung substituierten Phthaliden sowie dabei anfallende neue Benzylderivate**

Gegenstand der vorliegenden Erfindung ist ein neues Verfahren, bei dem, je nach Reaktionsführung, Dibenzyl-2-carbonsäuren oder in 3-Stellung durch einen, gegebenenfalls substituierten, Benzylrest substituierte Phthalide erhalten werden. Einige der nach dem neuen Verfahren hergestellten Verbindungen sind bisher unbekannt gewesen.

Es ist bekannt, Dibenzyl-2-carbonsäure auch als 2-(2-Phenylethyl)-benzoesäure bezeichnet, aus 3-Benzalphthalid durch Reduktion mit Zink oder katalytische Hydrierung herzustellen. Das für diese Verfahren benötigte Benzalphthalid wird in einem Schmelzprozeß bei ca. 250 °C aus Phthalsäureanhydrid und Phenylessigsäure gewonnen. Diese Arbeitsweise ist umständlich, zeitraubend und technisch nur mit einem großen Aufwand realisierbar.

Es ist weiterhin auch bekannt, daß beim Umsetzen von Phthalid mit Benzaldehyd im alkalischen Medium 3-(α-Hydroxybenzyl)-phthalid entsteht. Eine direkte Umwandlung dieses Produktes in Benzalphthalid war bisher jedoch nicht gelungen; vielmehr mußte der technisch sehr umständliche Weg über das in α-Stellung acetylierte Produkt 3-(α-Acetoxybenzyl)-phthalid genommen werden. (Vgl. J.Org.Chem. 1962, S.1602 ff.)

Es bestand deshalb die Aufgabe, ein Verfahren zu finden, mit dessen Hilfe es möglich ist, die Reaktion von Phthalid mit, gegebenenfalls substituierten, Benzaldehyden so zu leiten, daß direkt die durch einen Benzylrest in 3-Stellung substituierten Phthalide oder die entsprechenden Dibenzyl-2-carbonsäuren entstehen.

In Erfüllung dieser Aufgabe wurde nun ein Verfahren zur Herstellung von Dibenzyl-2-carbonsäuren und/oder in 3-Stellung durch einen, gegebenenfalls substituierten, Benzylrest substituierten Phthaliden gefunden, bei dem Phthalid mit aromatischen Aldehyden im alkalischen Medium umgesetzt werden, das dadurch gekennzeichnet ist, daß man das Reaktionsprodukt der alkalischen Umsetzung in Gegenwart von an sich bekannten Hydrierungskatalysatoren mit Wasserstoff bei einem Druck zwischen 50 und 200 bar umsetzt und anschließend das gewünschte Hydrierungsprodukt isoliert.

Bei der erfindungsgemäßen Verfahrensweise erfolgt in einem ersten Schritt die Anlagerung des Aldehyds an das Phthalid. Dieser Schritt muß im wasserfreien und alkalischen Medium durchgeführt werden. Er läuft bereits bei Raumtemperatur ab. Im allgemeinen wird dabei so vorgegangen, daß man das Phthalid in einem geeigneten Lösungsmittel, in dem das Alkali gelöst vorliegt, auflöst, die Lösung auf Raumtemperatur abkühlt und anschließend den Aldehyd so zufügt, daß die Temperatur des Gemisches möglichst nicht über 30 °C

ansteigt. Anschließend läßt man in diesem Temperaturbereich nachreagieren.

Als Lösungsmittel werden bevorzugt Alkohole eingesetzt, vorzugsweise Methanol oder Ethanol.

Die anschließende Hydrierung findet unter Druck bei erhöhter Temperatur statt. Eine Abtrennung des Reaktionsproduktes des ersten Schrittes ist nicht notwendig. Der anzuwendende Druck kann zwischen 50 und 200 bar schwanken; vorzugsweise liegt er zwischen 80 und 150 bar. Die Hydriertemperatur kann zwischen 40 und 150 °C liegen; vorzugsweise wird im Temperaturbereich zwischen 50 und 110 °C hydriert.

Als Hydrierkatalysatoren können die bekannten Hydrierkatalysatoren eingesetzt werden. Als Beispiele seien die Edelmetalle der VIII. Gruppe des Periodensystems genannt, die entweder als Metallpulver oder auf einem geeigneten Trägermaterial wie z.B. $Al_2O_3$ oder Kohle aufgebracht eingesetzt werden. Bevorzugter Hydrierkatalysator ist metallisches Palladium.

Es ist empfehlenswert, die Hydrierung in Gegenwart von geringen Mengen Wasser durchzuführen. Damit wird erreicht, daß die mögliche Umlagerung des Reaktionsproduktes aus der ersten Stufe zu gegebenenfalls substituiertem 2-Phenylindandion-1,3 unterdrückt wird. Diese Umlagerung findet bei erhöhten Temperaturen überwiegend im wasserfreien Medium statt. Es genügt deshalb nur eine geringe Menge an Wasser, um diese Nebenreaktion zu unterdrücken. Sie liegt in der Größenordnung von 0,1 bis 10 Vol.-%, vorzugsweise 0,5 bis 6 Vol.-%, bezogen auf das Gesamtvolumen an Lösungsmittel.

Bei vollständiger Hydrierung entstehen Dibenzyl-o-carbonsäuren bzw. deren Alkalisalze, die durch folgende allgemeine Formel I gekennzeichnet werden:

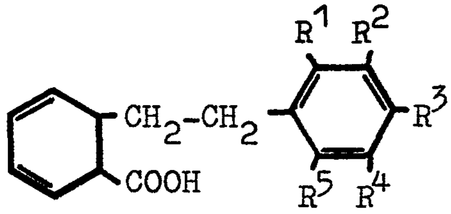

,

in der die Reste $R^1$ bis $R^5$ gleiche oder ungleiche Substituenten aus der Gruppe Wasserstoff, Halogen, Alkyl, Alkoxyl, Aryl, Aralkyl, Carboxyl, Carboalkoxyl oder der Aminorest ist, $R^1$ und $R^2$ können auch Bestandteil eines ankondensierten aromatischen Rings und $R^3$ kann auch ein Rest der Formel

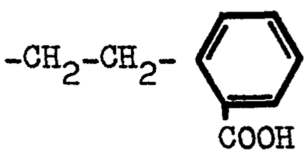

sein. Die Alkylreste haben bevorzugt 1 bis 4 C-Atome.

Wenn die Hydrierung nicht vollständig durchgeführt wird, entstehen als Zwischenprodukte substituierte Phthalide der Formel

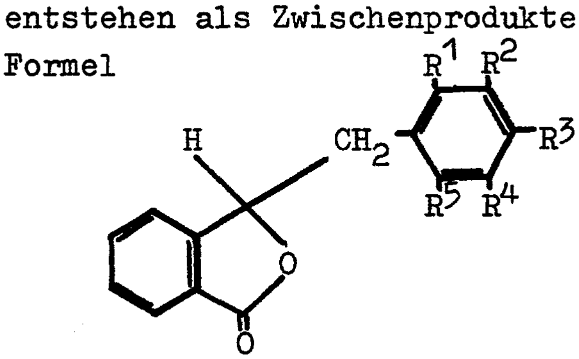

in der die Reste $R^1$ bis $R^5$ die gleiche Bedeutung wie oben besitzen. Diese Verbindungen können bei vorzeitigem Abbruch der Hydrierungsreaktion in gleicher Weise wie die vollständigen Hydrierungsprodukte aus dem Reaktionsgemisch abgetrennt werden.

Die Abtrennung der gewünschten Reaktionsprodukte erfolgt im allgemeinen in der Weise, daß man das Lösungsmittel abdestilliert, den Rückstand mit Wasser aufnimmt und darauf die gewünschte Säure durch Zusatz einer Mineralsäure ausfällt.

Die gemäß vorliegenden Verfahren erhältlichen Dibenzylorthocarbonsäuren und deren Substitutionsprodukte sind wertvolle Zwischenprodukte zur Herstellung von, gegebenenfalls substituiertem 10,11-Dihydro-5H-dibenzo [a,d] cyclohepten-5-on, das unter dem Trivialnamen Dibenzosuberon ein bekanntes Ausgangsprodukt zur Herstellung von Pharmazeutika, wie z.B. Antidepressiva, ist. Weiterhin lassen sich die gemäß dem vorliegenden Verfahren erhaltenen Produkte auch als Vorprodukte für die Polymerchemie einsetzen.

Beispiel 1

2-(2-Phenylethyl)-benzoesäure (= Dibenzyl-o-carbonsäure)

In einem 1 Liter Dreihalskolben, der mit Rührer, Tropftrichter und Innenthermometer ausgerüstet war, wurden unter Stickstoff 11,5 g (0,5 g-Atom) metallisches Natrium in 400 ml absolutem Methanol gelöst. Danach wurden in der erhaltenen Methylatlösung bei 35 °C 67,1 g (0,5 Mol) Phthalid gelöst. Nach Abkühlen auf 23 bis 24 °C wurden unter Stickstoff und Rühren in die Lösung 53 g (0,5 Mol) frisch destillierter Benzaldehyd in dem Maße eindosiert, daß die Temperatur des Kolbeninhaltes nicht über 25 bis 26 °C anstieg. Hierzu war eine Dosierzeit von 15 bis 20 Minuten erforderlich. Zur Nachreaktion wurde 2 bis 3 Stunden bei 25 bis 26 °C gerührt.

Die Kondensationslösung wurde sodann in einen 1 Liter Magnethub-Autoklaven überführt, mit 10 g Palladiumkatalysator (5 % Pd auf Kohle) versetzt und unter einem Wasserstoffdruck von 100 bar 6 bis 7 Stunden bei 90 bis 95 °C

hydriert. Danach wurde der abgekühlte und entspannte Autoklaveninhalt filtriert und aus dem farblosen Filtrat das Methanol abdestilliert.

Der ölige Destillationsrückstand wurde in 1.400 ml Wasser aufgenommen, mit konzentrierter Salzsäure (etwa 50 ml) unter Rühren auf pH 1 angesäuert und darauf die farblose und mikrokristallin ausgefallene Dibenzyl-o-carbonsäure abfiltriert und mit Wasser bis zu Chloridfreiheit gewaschen. Nach Trocknen bei 70 bis 100 °C im Wasserstrahlvakuum wurden 92,7 g, entsprechend 82 % Ausbeute, einer Rohsäure vom Schmp.: 113 bis 126 °C erhalten. Die Rohsäure läßt sich aus Cyclohexan (1 g aus 12 bis 15 ml), oder aus Toluol (1 g aus 3 bis 4 ml) umkristallisieren. Bezogen auf eingesetztes Phthalid wurden aus Cyclohexan 84 g (74,3 % Ausbeute) reine Säure erhalten. Farblose Nadeln vom Schmelzpunkt: 129 bis 132 °C.

Beispiel 2

2-(2-Phenylethyl)-benzoesäure

In einem 2 Liter-Kolben, der wie im Beispiel 1 ausgerüstet war, wurden 800 ml 8,0 Gew.-%ige technische Natriummethylatlösung (1,25 Mol $NaOCH_3$/Liter) vorgelegt, auf 40 bis 45 °C gebracht und bei dieser Temperatur 134,2 g (1 Mol) Phthalid unter Rühren eingetragen. Nach dessen Auflösung wurde die Luft im Kolben durch Stickstoff verdrängt, die Lösung auf 23 bis 24 °C abgekühlt und im Verlaufe von 20 bis 30 Minuten 106 g (1 Mol) Benzaldehyd bei 25 bis 26 °C nicht überschreitender Temperatur zugetropft. Bei dieser Temperatur wurde 2,5 Stunden nachreagieren gelassen. Danach wurde die Lösung in einen Edelstahlautoklaven eingefüllt, mit 20 g Katalysator (5 % Pd auf Kohle), der mit 20 ml Wasser angerührt war, versetzt und wie im Beispiel 1 hydriert und aufgearbeitet. Es wurden 176,3 g (78 % Ausbeute) an farbloser Rohsäure vom Schmelzpunkt 126 bis 129 °C erhalten. Die Umkristallisation des Rohproduktes ergab ein reines Produkt mit einem Schmelzpunkt von 131 bis 134 °C.

Mit Natriumethylatlösung, statt der Natriummethylatlösung, wurde ein gleichartiges Ergebnis erhalten.

<u>Beispiel 3</u>

<u>Dibenzyl-2,4'-dicarbonsäure</u>

Wie in Beispiel 1 wurden anstelle von Benzaldehyd 82 g Terephthalaldehydsäuremethylester eingesetzt und die Verfahrensweise des Beispiels 1 wiederholt.

Vor dem Ansäuern der hydrierten Kondensationslösung wurde jedoch die wässrige Lösung nach Zugabe von 0,5 Mol NaOH als ca. 30 %ige Lauge 1,5 Stunden auf 95 °C (Verseifung des Esters) erhitzt. Das danach durch Ansäuern ausgefällte und abfiltrierte Produkt wurde aus Eisessig umkristallisiert. 85 g (= 63 % Ausbeute) an Dibenzyl-2,4'-dicarbonsäure vom Schmelzpunkt: 241 bis 243 °C wurden erhalten. Die Struktur dieser bisher nicht bekannten Verbindung wurde durch Massebestimmung, sowie durch IR- und NMR-spektroskopische Untersuchungen bewiesen.

<u>Beispiel 4</u>

<u>1,4-Bis-(β-phenylethyl)-benzol-2'. 2''-dicarbonsäure</u>

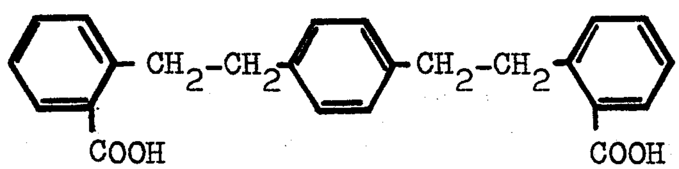

In einem 2 Liter Dreihalskolben wurden gemäß der Verfahrensweise von Beispiel 2 800 ml 8,0 Gew.-%ige Natriummethylatlösung mit 134,2 g (1 Mol) Phthalid und 67 g (0,5 Mol) Terephthaldialdehyd umgesetzt. Dabei wurde der

Aldehyd in kristallisiertem Zustand portionsweise innerhalb von 20 Minuten in die Phthalidlösung eingetragen.

Die Hydrierlösung war nach Abfiltrieren des Pd-Katalysators völlig farblos. Analog der in Beispiel 1 beschriebenen Aufarbeitung wurden 146 g einer farblosen, pulvrigen Rohsäure vom Schmelzpunkt: 152 bis 188 °C erhalten, die nach GC-Analyse etwa 72 % des Zielproduktes enthielt. Nach zweimaliger Umkristallisation aus Methylglykol (1 g aus 5 bis 6 ml) wurden 58 g reine Dicarbonsäure in Form farbloser Nadeln vom Schmelzpunkt: 218 bis 221 °C erhalten. Die Struktur dieser bisher nicht bekannten Substanz wurde durch Massebestimmung, sowie durch IR- und NMR-spektroskopische Untersuchungen bestätigt.

Elementaranalyse: $C_{24}H_{22}O_4$ (374,45).

Berechnet: C 76,98 %; H 5,92 %; O 17,09 %.

Gefunden: C 77,22 %; H 6,11 %; O 16,92 %.

<u>Beispiel 5</u>

<u>2-[2-(4-Tolyl)-ethyl]-benzoesäure</u>

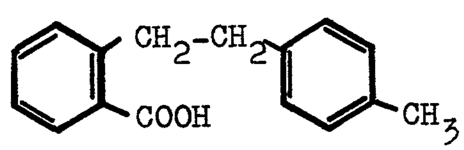

Nach der Verfahrensweise von Beispiel 1 wurde statt Benzaldehyd 60 g p-Tolylaldehyd mit Phthalid kondensiert und die Reaktionslösung hydriert.

Das Filtrat der Hydrierlösung lieferte nach der Aufarbeitung 88,6 g einer klumpigen, leicht klebrigen, farblosen Fällung vom Schmelzpunkt: 48 bis 82 °C. Aus Petrolether (Sdp.: 50 bis 80 °C) im Verhältnis 1 g zu 4 ml umkristallisiert, wurde die reine Säure vom Schmelzpunkt: 82 bis 84 °C erhalten.
Nach Literaturangaben beträgt der Schmelzpunkt 83 bis 84 °C nach Umkristallisation aus Ethanol.

Beispiel 6

2-[2-(4-Methoxyphenyl)-ethyl]-benzoesäure

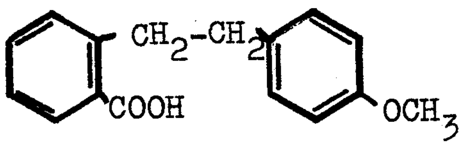

Es wurde analog Beispiel 2 gearbeitet, jedoch statt Benzaldehyd 136 g (1 Mol) Anisaldehyd eingesetzt. Nach der Aufarbeitung wurden 92 g farblose Rohsäure vom Schmelzbereich 67 bis 98 °C erhalten. Aus Cyclohexan (1 g aus 4 ml) umkristallisiert, wurden 63 g nadelförmiges Kristallisat vom Schmelzpunkt: 117 bis 119 °C gewonnen.

Gemäß Literaturangaben beträgt der Schmelzpunkt: 118 bis 120 °C nach Umkristallisation aus 60 %igem Ethanol.

Beispiel 7

1-(α-Naphthyl)-2-(o-carboxyphenyl)-ethan

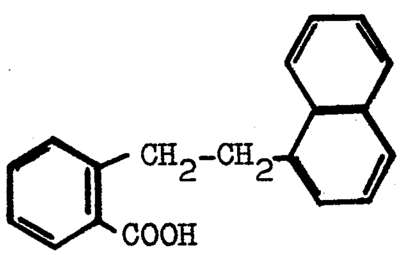

In der Apparatur von Beispiel 2 wurden 134,2 g (1 Mol) Phthalid bei 35 °C in 782 ml einer Natriummethylatlösung gelöst, die 1,28 Mol $NaOCH_3$/Liter enthielt. In die auf 25 bis 26 °C abgekühlte Lösung wurden sodann nach Verdrängen der Luft durch Stickstoff unter Rühren 156,2 g (1 Mol)

1-Naphthaldehyd innerhalb von 20 Minuten eingetropft, wobei die Temperatur des Reaktionsgemisches bei 25 bis 26 °C gehalten wurde. Bei dieser Temperatur wurde 3 Stunden gerührt. Darauf wurde die Kondensationslösung nach Zugabe von 25 ml Wasser in Gegenwart von 20 g Palladium-Kohle-Katalysator (Pd-Gehalt 5 Gew.-%) 9 Stunden unter 100 bar Wasserstoffdruck bei 100 °C hydriert.

Die Aufarbeitung der Hydrierlösung, wie in Beispielen 1 und 2 vorbeschrieben, lieferte 146 g Rohsäure vom Schmelzbereich 108 bis 136 °C. Aus Cyclohexan (1 g auf 25 ml) wurde umkristallisiert.

Es wurden 103 g reine, in Nadeln anfallende Säure vom Schmelzpunkt: 145 bis 148 °C, deren Struktur durch IR- und NMR-Spektroskopie bestätigt wurde, erhalten.

Elementaranalyse: $C_{19}H_{16}O_2$ (276,34).

Berechnet: C 82,58 %; H 5,84 %; O 11,58 %.

Gefunden: C 82,39 %; H 5,78 %; O 11,47 %.

Beispiel 8

(3-Phthalidyl)-(α-naphthyl)-methan

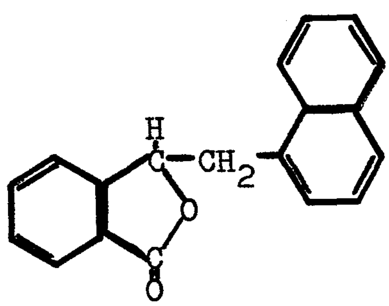

Die Arbeitsweise des Beispiels 7 wurde wiederholt, jedoch wurde die Hydrierung mit 20 g Palladium auf $Al_2O_3$ (Pd-Ge-

halt 5 %), statt Pd auf Kohle, durchgeführt. Nachdem die Hälfte der in Beispiel 7 verwendeten Wasserstoffmenge aufgenommen war, wurde die Hydrierung abgebrochen und das Rohprodukt entsprechend Beispiel 7 aufgearbeitet.

Die Aufarbeitung lieferte 76 % Ausbeute an Phthalidylnaphthylmethan, dessen Struktur durch IR- und NMR-Untersuchung bestätigt wurde. Nach Umkristallisation aus Methanol (1 g auf 10 ml) wurden farblose Nadeln vom Schmelzpunkt: 115 bis 118 °C erhalten.

Elementaranalyse: $C_{19}H_{14}O_2$ (274,32).

Berechnet: C 83,19 %; H 5,14 %; O 11,67 %.

Gefunden: C 83,08 %; H 5,06 %; O 11,75 %.

<u>Beispiel 9</u>

<u>3-Benzyl-phthalid</u>

Die Arbeitsweise des Beispiels 2 wurde unter den dort angegebenen Bedingungen wiederholt mit dem Unterschied, daß als Katalysator Palladium auf $Al_2O_3$, statt Pd auf Kohle, eingesetzt und die Hydrierung auf der Stufe des Benzylphthalids abgebrochen wurde. Nach Aufarbeitung wurde diese Substanz in einer Ausbeute von 81 % erhalten.

Troisdorf, den 10.3.1982
OZ: 82021 (4122) Dr.Sk/Ce

Patentansprüche:

1. Verfahren zur Herstellung von Dibenzyl-2-carbonsäuren und/oder in 3-Stellung durch einen, gegebenenfalls substituierten, Benzylrest substituierten Phthaliden, bei dem Phthalid mit aromatischen Aldehyden im alkalischen Medium umgesetzt wird, dadurch gekennzeichnet, daß man das Reaktionsprodukt der alkalischen Umsetzung in Gegenwart von an sich bekannten Hydrierungskatalysatoren mit Wasserstoff bei einem Druck zwischen 50 und 200 bar umsetzt und anschließend das gewünschte Hydrierungsprodukt isoliert.

2. Verfahren gemäß Anspruch 1, dadurch gekennzeichnet, daß die Hydrierung bei Temperaturen zwischen 40 und 150 °C durchgeführt wird.

3. Verfahren gemäß Anspruch 1 oder 2, dadurch gekennzeichnet, daß die Hydrierung in Gegenwart von geringen Mengen Wasser durchgeführt wird.

4. 1,4-Bis-(β-phenylethyl)-benzol-2', 2''-dicarbonsäure der Formel

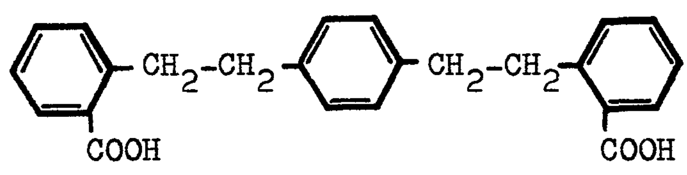

5. 1-(α-Naphthyl)-2-(o-carboxyphenyl)-ethan der Formel

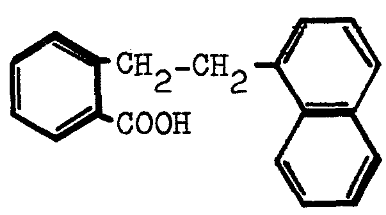

6. (3-Phthalidyl)-(α-naphthyl)-methan der Formel

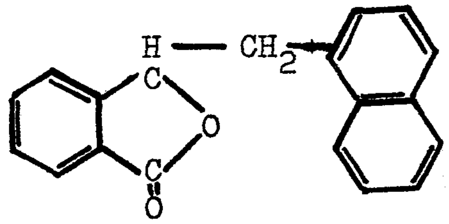

7. Dibenzyl-2,4'-dicarbonsäure der Formel

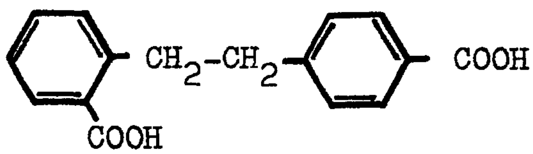

 Troisdorf, den 10.3.1982

OZ: 82021 (4122) Dr.Sk/Ce

Patentansprüche: (für Bestimmungsland Österreich)

1. Verfahren zur Herstellung von Dibenzyl-2-carbonsäuren und/oder in 3-Stellung durch einen, gegebenenfalls substituierten, Benzylrest substituierten Phthaliden, bei dem Phthalid mit aromatischen Aldehyden im alkalischen Medium umgesetzt wird, dadurch gekennzeichnet, daß man das Reaktionsprodukt der alkalischen Umsetzung in Gegenwart von an sich bekannten Hydrierungskatalysatoren mit Wasserstoff bei einem Druck zwischen 50 und 200 bar umsetzt und anschließend das gewünschte Hydrierungsprodukt isoliert.

2. Verfahren gemäß Anspruch 1, dadurch gekennzeichnet, daß die Hydrierung bei Temperaturen zwischen 40 und 150 °C durchgeführt wird.

3. Verfahren gemäß Anspruch 1 oder 2, dadurch gekennzeichnet, daß die Hydrierung in Gegenwart von geringen Mengen Wasser durchgeführt wird.

4. Verfahren zur Herstellung von 1,4-Bis-(ß-phenylethyl)-benzol-2',2"-dicarbonsäure gemäß einem der Ansprüche 1 bis 3, dadurch gekennzeichnet, daß man das im alkalischen Medium erhaltene Umsetzungsprodukt von Phthalid und Terephthalaldehyd hydriert.

5. Verfahren zur Herstellung von Dibenzyl-2,4'-dicarbonsäure gemäß einem der Ansprüche 1 bis 3, dadurch gekennzeichnet, daß man das im alkalischen Medium erhaltene Umsetzungsprodukt von Phthalid und Terephthalaldehydsäuremethylester hydriert, das Hydrierungsprodukt verseift und anschließend die gewünschte Säure durch Zusatz einer Säure ausfällt.

0090196

6. Verfahren zur Herstellung von (3 Phthalidyl)-(α-naphthyl)-methan und 1-(α-Naphthyl)-2-(o-carboxyphenyl)-ethan gemäß einem der Ansprüche 1 bis 3, dadurch gekennzeichnet, daß man das im alkalischen Medium erhaltene Umsetzungsprodukt von Phthalid und 1-Naphthaldehyd mit der zur Bildung des gewünschten Endprodukts notwendigen Menge Wasserstoff hydriert.

Europäisches Patentamt

**EUROPÄISCHER RECHERCHENBERICHT**

0090196 Nummer der Anmeldung

EP 83 10 2110

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-1 163 791 (MERCK) * Anspruch; Spalte 2 - Spalte 3, Zeile 22 * | 1-3 | C 07 C 63/00<br>C 07 C 51/09<br>C 07 D 307/88 |
| Y | DD-A- 98 909 (TENOR et al.) * Anspruch * | 1-2 | |
| X | US-A-2 677 703 (TOLAND) * Anspruch 2; Spalte 3, Zeilen 49-53, 63-66 * | 7 | |
| A | CH-A- 407 975 (HOFFMANN-LA ROCHE) * Anspruch * | 1 | |
| | | | **RECHERCHIERTE SACHGEBIETE (Int. Cl. 3)** |
| | | | C 07 C 63/00<br>C 07 C 51/00<br>C 07 D 307/00<br>C 07 D 315/00 |

Der vorliegende Recherchenbericht wurde für alle Patentansprüche erstellt.

| Recherchenort | Abschlußdatum der Recherche | Prüfer |
|---|---|---|
| DEN HAAG | 10-06-1983 | KLAG M.J. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82